# EUROPÄISCHE PATENTSCHRIFT

(11) **EP 1 501 457 B1**
(45) Veröffentlichungstag und Bekanntmachung des Hinweises auf die Patenterteilung: **14.11.2007**
(21) Anmeldenummer: 03724879.6
(22) Anmeldetag: 02.05.2003
(51) Int. Cl.: A61F 5/055, A61F 5/01, B64D 10/00

(54) **EINRICHTUNG ZUM ENTLASTEN DER HALSWIRBELSÄULE**
DEVICE FOR SUPPORTING THE CERVICAL VERTEBRAL COLUMN
DISPOSITIF POUR SOULAGER LA COLONNE CERVICALE

(30) Priorität: 03.05.2002 DE 10219868
(43) Veröffentlichungstag der Anmeldung: 02.02.2005
(73) Patentinhaber: Schmitz, Josef, 83083 Riedering (DE)
(72) Erfinder: Schmitz, Josef, 83083 Riedering (DE)
(74) Vertreter: von Puttkamer, Nikolaus
(86) Internationale Anmeldenummer: PCT/DE2003/001414
(87) Internationale Veröffentlichungsnummer: WO 2003/092561

(56) Entgegenhaltungen:
- WO-A-01/25088
- DE-A- 3 905 115
- FR-A- 2 700 746
- US-A- 5 039 035
- US-A- 5 752 927

## Beschreibung

Die vorliegende Erfindung betrifft eine Einrichtung zum Entlasten der Halswirbelsäule nach dem Oberbegriff des Patentanspruches 1.

Aus der Druckschrift WO 01/25088 geht eine derartige Einrichtung hervor, die im wesentlichen eine Druckpolstereinrichtung aufweist, die in Bezug auf den Hals des Benutzers gegenüberliegend wenigstens zwei vordere Druckpolster aufweist, die sich einerseits an der Schulter eines Benutzers und andererseits an den Unterseiten der seitlichen Unterkieferbereiche des Kopfes des Benutzers abstützen. Ferner weist die bekannte Einrichtung wenigstens ein weiteres hinteres Druckpolster auf, das sich einerseits an der Schulter des Benutzers und andererseits an der Hinterhauptschuppe des Kopfes des Benutzers abstützt. Diese drei Druckpolster bewirken in ihrer Wirkrichtung einen Druck zur Abstützung des Kopfes in vertikaler Richtung. Eine Steuer- und Regeleinheit kann den Druck in den Druckpolstern selektiv erhöhen oder erniedrigen und je nach Kopfneigung, die beim Einwirken der durch eine Vorrichtung ermittelte g-Kraftkomponente gerade vorherrscht, unterschiedliche Gegenkräfte in der Wirkrichtung aufbauen.

Ein Problem einer derartigen Einrichtung zum Entlasten der Halswirbelsäule besteht z.B. darin, dass die beiden sich an den Unterseiten der seitlichen Unterkieferbereiche abstützenden vorderen Druckpolster auch im drucklosen Zustand bei nach vorne bewegtem Kopf eine Drehbewegung des Kopfes erschweren.

Zudem besteht die Möglichkeit, dass dann, wenn der Kopf unter größerer Kraftanwendung nach vorne geneigt wird, das Kinn des Benutzers zwischen den vorderen Bereichen der beiden vorderen Druckpolster hindurchrutscht, so dass die kraftkompensierende Funktion dann nicht mehr gewährleistet und es zudem nicht mehr möglich ist, Bewegungen zur Seite auszuführen.

Ein weiteres Problem der bekannten Einrichtung besteht darin, dass eine erhebliche Beeinträchtigung der Funktion der vorderen Druckpolster durch den Kinnriemen des Helms eines Benutzers gegeben ist. Ein Helm ist jedoch unverzichtbar.

Die Aufgabe der vorliegenden Erfindung besteht daher darin, eine Einrichtung zum Entlasten der Halswirbelsäule zu schaffen, die eine bessere Bewegungsfreiheit des Kopfes zulässt.

Diese Aufgabe wird durch eine Einrichtung zum Entlasten der Halswirbelsäule mit den Merkmalen des Patentanspruches 1 gelöst.

Der wesentliche Vorteil der vorliegenden Erfindung besteht darin, dass durch das vorgesehene Kinn-Auflageteil, das am Kinnriemen des Helmes befestigt ist, eine besonders gute Auflage des Kopfes mit dem Kinn auf dem Auflageteil einerseits und des Auflageteiles auf den vorderen Druckpolstern andererseits erreicht werden, wobei insbesondere der zwischen den vorderen Endbereichen der vorderen Druckpolster bestehende Spalt durch das Auflageteil überbrückt wird. Dadurch, dass das Auflageteil auf den vorderen Druckpolstern leicht gleiten kann, werden vorteilhafterweise Drehbewegungen des Kopfes ermöglicht. Ein schädliches Eindringen des Kopfes in den genannten Spalt zwischen den vorderen Endbereichen der vorderen Druckpolster bei starken Bewegungen des Kopfes nach vorne wird zuverlässig verhindert.

Weitere Vorteile der vorliegenden Erfindung bestehen darin, dass die im Falle von g-Belastungen die auf den Kopf mit Helm eines Piloten wirkenden Kräfte an dem Kinngelenk und der Hinterhauptschuppe teilweise kompensiert werden, wobei die vorderen Druckpolster am Kinngelenk die Kompensation bei einer Kopfneigung bis vorzugsweise 30° nach vorne und bis vorzugsweise mehr als 17° nach hinten übernehmen. Die hinteren Druckpolster an der Hinterhauptschuppe des Kopfes übernehmen die Kompensation bei einer Kopfneigung von vorzugsweise 22° bis vorzugsweise mehr als 40° nach hinten. Bei einer nahezu aufrechten Kopfhaltung von etwa 17° bis 22° nach vorne und nach hinten und etwa 5° seitlich wird der Kopf nicht unterstützt. Die Schwerpunkte des Kopf-/Helmsystems liegen dabei nahezu übereinander. Die g-relevanten Kräfte auf die Halswirbelsäule in dieser Kopfstellung sind daher moderat, da die Komponente durch Gegenzug der Nackenmuskulatur gering ist. Sie liegen bei 9 g in dieser Stellung ungefähr wie bei 4 g maximal. Die angegebenen Winkelgrade beziehen sich auf die Kopfstellung 0°, bei der der Schwerpunkt des Kopfes bzw. der Masse Helm/Kopf vertikal über dem Drehpunkt (Atlas) auf der Wirbelsäule liegt.

Während des Betriebes ohne g-Belastung wird bei einer Kopfneigung durch geringen Druck in den Druckpolstern ein Kraftschluss der Druckpolster und dem Kopf aufrecht erhalten. Die nahezu volle Bewegungsfreiheit ist dabei bei geringem Gegenhaltedruck gewährleistet.

Bei einer g-Belastung bis 6 g werden der Betrag und der Vektor der Belastung in Relation zur Kopfhaltung im Moment dieser Einwirkung durch einen g-Sensor erfasst und dem daraus resultierenden gegenzusteuernden Druckpolster eine Gegenkraft durch eine Steuer- und Regeleinheit vorgegeben. Ein jeweils zuständiges Regelventil beaufschlagt das entsprechende Druckkissen, bis der vorgegebene Auflagedruck am Kopf erreicht ist. Durch Kraftsensoren auf der Oberfläche der Druckpolster wird die Kraft auf den Kopf erfasst. Diese Kraft wird mit einer Sollvorgabe der Steuer- und Regeleinheit verglichen und eventuell wird eine Druckminderung im Druckpolster durch das Ansteuern des entsprechenden Regelventils erreicht. Bei beabsichtigter Bewegung des Kopfes wird durch die Muskulatur ein zusätzlicher geringer Druck auf die Kraftsensoren aufgebracht, die Steuer- und Regeleinheit steuert den Druck zurück und lässt so eine Bewegung in der beabsichtigten Richtung zu. Bei einer Beschleunigung über 6 g werden die Regelventile zu den Druckpolstern für maximale Druckbeaufschlagung geöffnet und dann komplett geschlossen. Der Kopf wird bei diesen Belastungen gestützt. Eine' geregelte Bewegungsfreiheit wird dabei nicht angestrebt, da dies physisch nicht machbar ist. Die Wirkungsweise entlastet je nach Kopfstellung und Schwerpunktlage vorteilhafterweise die Belastung der Wirbelsäule bis ca. 60% und die Beanspruchung der Nackenmuskulatur bis nahezu 100%.

Normalerweise sind bei einer hohen g-Belastung Schädigungen der Halswirbelsäule durch eine resultierende Kraft, die bis zum 2,0fachen der g-Kraft aus dem Gewicht beträgt, zu erwarten. Solche Schädigungen können in vorteilhafter Weise durch die erfindungsgemäße Einrichtung vermieden werden. Ebenso können durch die Erfindung Belastungen der Nackenmuskulatur vermieden werden, die normalerweise Ermüdungen, Blockierungen, neurologische Defizite, Koordinationsstörungen, Kopfschmerzen, Teilleistungsstörungen und eine verringerte Konzentrationsfähigkeit nach sich ziehen können.

Ein weiterer wesentlicher Vorteil der vorliegenden Erfindung besteht auch darin, dass durch das Helm-Display angezeigte Bilder und Informationen (Helmet Mounted Display) vibrationsfrei durch den Piloten gesehen werden können, weil durch die vorliegende Einrichtung das Kopf-Helm-System stabilisiert wird.

Vorteilhafte Ausgestaltungen der Erfindung gehen aus den Unteransprüchen hervor.

Im folgenden werden die Erfindung und deren Ausgestaltungen im Zusammenhang mit den Figuren näher erläutert. Es zeigen:
- Fig. 1: zur Erläuterung der Erfindung eine schematische Darstellung, in der der Schwerpunkt des Kopfes, der Drehpunkt zwischen Halswirbelsäule und Kopf sowie der Angriffspunkt der Nackenmuskulatur eingetragen sind;
- Fig. 2: eine perspektivische Darstellung einer bevorzugten Ausführungsform der erfindungsgemäßen Einrichtung, im wesentlichen bestehend aus dem am Kinnriemen eines Helmes befestigten Auflageteil, den vorderen Druckpolstern mit harter Oberfläche für den Kraftschluss mit dem Kopfteil und dem Schulterteil mit Auflageschale;
- Fig. 3: ein schematisches Blockschaltbild der Regel- und Steuereinheit zur Erläuterung der Steuerung des Druckaufbaus sowie des Druckabbaus in einem Druckpolster; und
- Fig. 4: eine Weiterbildung der Erfindung..

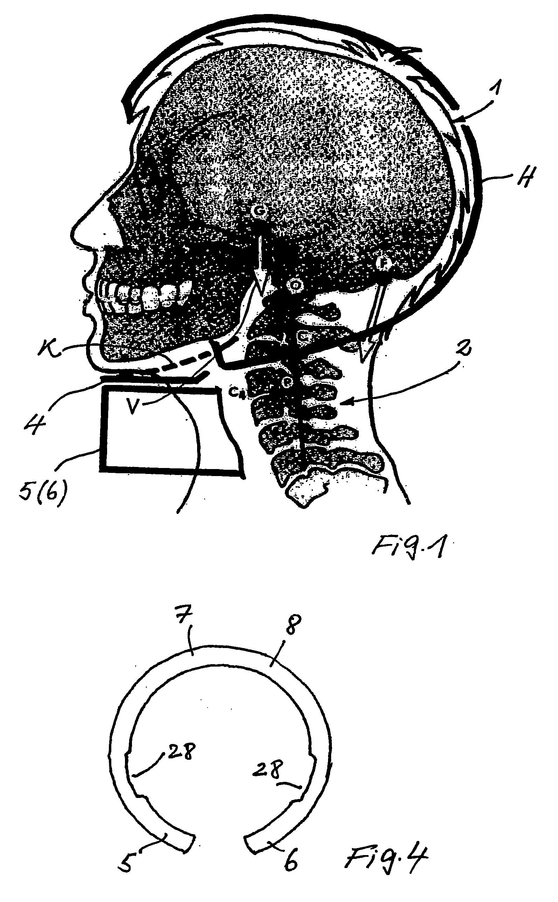

Zu der Erfindung führten die folgenden Überlegungen. Gemäß Figur 1 wird der Kopf 1 eines Piloten in Bezug auf die Halswirbelsäule 2 um den Drehpunkt O verdreht, der sich am oberen Ende der Halswirbelsäule 2 befindet. Die Drehung erfolgt durch die Nackenmuskulatur, die im Punkt F am Kopf 1 angreift. In der Gegenrichtung wirkt das Kippmoment des Kopfes 1, das sich aus dessen Gewicht ergibt und versucht den Kopf 1 um den Drehpunkt O zu drehen. Es ist erkennbar, dass beim Tragen eines Helmes, insbesondere dann, wenn an diesem zusätzliche Ausrüstungsgegenstände, wie beispielsweise Visier- oder Nachsichtgeräte angebracht sind, das Gewicht des Kopfes 1 und somit auch das Kippmoment um den Drehpunkt O stark vergrößert werden. Der entsprechende Vektor ist in der Figur 1 mit V bezeichnet. Um nun zu verhindern, dass bei einer großen g-Belastung und beim Neigen des Kopfes 1 nach vorne der Kopf 1 durch die vorderen Endbereiche der vorderen Druckpolster hindurch gedrückt wird, so dass die Funktion derselben nicht mehr gegeben ist, wird erfindungsgemäß am Helm H, vorzugsweise am Gurtteil K des Helmes H an der den vorderen Druckpolstern 5, 6 zugewandten Seite ein Auflageteil 4 angebracht, das bei der in der Figur 1 dargestellten normalen Kopfhaltung ohne g-Belastung bei einer Kopfneigung bei einem geringen Druck in den vorderen Druckpolstern 5, 6 auf der Oberfläche derselben gleiten kann, wobei ein Kraftschluss der vorderen Druckpolster 5, 6 mit dem Kopf 1 aufrecht erhalten wird. Drehbewegungen des Kopfes 1 sind dabei gut möglich. Eine nahezu völlige Bewegungsfreiheit ist dabei mit geringem Gegenhaltedruck gewährleistet.

Bei einer hohen g-Belastung (bis 6 g) werden der Betrag und der Vektor der Belastung in Relation zur Kopfhaltung im Moment dieser Einwirkung durch Sensoren erfasst und gibt die Steuer- und Regeleinheit dem daraus resultierenden gegenzusteuernden Druckpolster eine Gegenkraft vor, bis ein vorgegebener Auflagedruck am Kopf 1 erreicht ist. Bei einer beabsichtigten Bewegung des Kopfes 1 wird durch die Muskulatur ein zusätzlicher geringer Druck auf die Kraftsensoren des jeweiligen vorderen Druckpolsters 5, 6 aufgebracht, so dass die Steuer- und Regeleinheit den Druck zurücksteuern und somit eine Bewegung in der beabsichtigten Richtung zulassen kann. Bei sehr großen Beschleunigungen (über 6 g) werden die Druckpolster für eine maximale Druckbeaufschlagung beaufschlagt, so dass der Kopf 1 bei diesen Belastungen voll gestützt wird. Eine geregelte Bewegungsfreiheit wird dabei nicht angestrebt, da dies physisch nicht machbar erscheint. Durch die Wirkungsweise der Druckpolster sowie des darauf gleitbar aufliegenden Auflageteiles 4 wird je nach Kopfstellung und Schwerpunktlage eine Verminderung der Belastung der Halswirbelsäule 2 bis ca. 60%, und der Beanspruchung der Muskulatur bis nahezu 100% erreicht.

Gemäß Figur 2 sind die vorderen Druckpolster 5, 6 sowie die hinteren Druckpolster 7, 8 am Randbereich einer oberen Öffnung 12 einer auf dem Nacken- und Schulterbereich eines Piloten aufliegenden Auflageschale 10 befestigt. Die Auflageschale 10 weist vorderseitig eine nach unten verlaufende schlitzförmige Öffnung 14 auf, die zum Anlegen der Auflageschale 10 öffenbar ist, und danach mit der Hilfe von wenigstens einer in Querrichtung verlaufenden Schließeinrichtung 16, bei der es sich beispielsweise um ein Klettverschlussteil handelt, durch Zuziehen der schlitzförmigen Öffnung 14 befestigbar ist.

Die hinteren Druckpolster 7, 8 liegen oberseitig in an sich bekannter Weise an der Hinterhauptschuppe des Kopfes 1 an. Die vorderen Druckpolster 5, 6 weisen an ihren Oberseiten integrierte Versteifungselemente 18, 20 auf, auf denen sich das Auflageteil 4 abstützen kann. Jedes der Druckpolster 5, 6, 7, 8 ist mit der Hilfe eines Fluids, insbesondere eines Gels, befüllbar, wie dies später näher erläutert werden wird, so dass es sich in der Wirkrichtung zum Kopf 1 des Piloten ausdehnen kann, um diesen zu stützen. Es wird darauf hingewiesen, dass die Druckpolster 5, 6, 7, 8 vorzugsweise durch Schaumpolster nach außen, innen und nach oben abgepolstert sein können.

Wenn die Auflageschale 10 auf dem Schulterbereich eines Piloten aufliegt und die Schließeinrichtung 16 verschlossen ist, liegen die Oberseiten der hinteren Druckpolster 7, 8 an der Hinterhauptschuppe des Kopfes 1 des Piloten an und sind die Oberseiten der vorderen Druckpolster 5, 6 mit ihren Versteifungselementen 18, 20 den seitlichen Unterkieferbereichen des Piloten zugewandt. Das Auflageteil 4 ist an Kinngurtteilen 22, 24 des in der Figur 2 nicht dargestellten Helmes H befestigt. Es ist dabei so gestaltet, dass es im normalen Zustand auf den Oberseiten der Versteifungselemente 18, 20 bzw. der diese abdeckenden Umhüllung der vorderen Druckpolster 5, 6 aufliegen kann, so dass es beim Drehen des Kopfes 1 des Piloten auf diesen frei gleiten kann. Ein Hindurchschieben des Kinnbereiches des Kopfes 1 wird dabei vermieden, weil das Auflageteil 4 mit seinem vorderen Bereich 4' den Spalt zwischen den vorderen Endbereichen der vorderen Druckpolster 5, 6 überbrückt. Vorzugsweise sind am vorderen Bereich 4' Seitenbereiche angeordnet bzw. angeformt, die sich ausgehend vom vorderen Bereich 4' schräg nach außen erstrecken und auf den vorderen Druckpolstern 5, 6 aufliegen, wenn der Kopf 1 eines Piloten geradeaus gerichtet ist. Die freien Endbereiche 4" der Seitenbereiche verlaufen vorzugsweise aus der Ebene der Seitenbereiche und des vorderen Bereiches 4' schräg nach oben, so dass die Gleitbewegung des Auflageteiles 4 nicht behindert werden kann.

An jedem Druckpolster 5, 6, 7, 8 ist oberseitig ein Kraftsensor 26 angeordnet, bei dem es sich beispielsweise um einen piezoresistiven Drucksensor handelt, der je nach dem auf ihn und auf das jeweilige Druckpolster ausgeübten Druck eine Ausgangsspannung zwischen z.B. 0 Volt und 10 Volt als Ausgangssignal ausgibt.

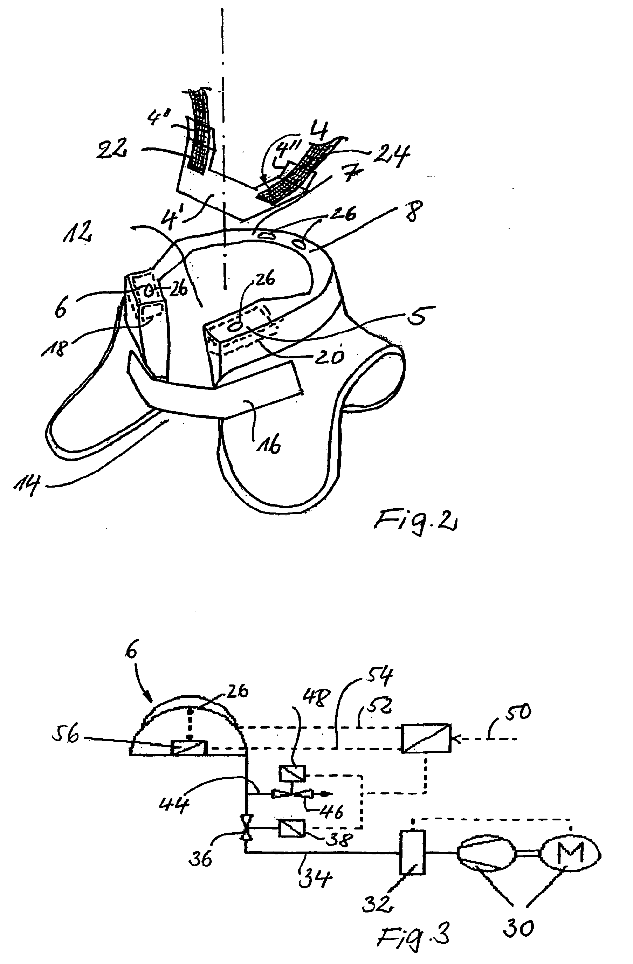

Die Figur 3 zeigt ein schematisches Blockschaltbild einer Steuer- und Regeleinheit zur Druckerzeugung und Druckbeaufschlagung der Druckpolster 5, 6, 7, 8, wobei in der Figur 3 beispielhaft nur ein Druckpolster 6 dargestellt ist. Ein Kompressor 30 erzeugt in einem Vorratsbehälter 32 den erforderlichen Betriebsdruck. Der Kompressor 30 ist über eine Leitung 34 und ein Regelventil 36 mit dem Druckpolster 6 zum Druckaufbau verbunden. Das Regelventil 36 wird über ein Stellglied 38 betätigt, das von der Elektronikeinheit 40 betätigt wird. In der Leitung 34 befindet sich zwischen dem Regelventil 36 und dem Druckpolster 6 eine Abzweigleitung 44 mit einem Ablassventil 46, über das der im Druckpolster 6 aufgebaute Druck bei seiner Betätigung durch das Stellglied 48, das ebenfalls von der Elektronikeinheit 40 angesteuert wird, nach außen druckentlastet wird.

Die Elektronikeinheit 40 empfängt von einem 3D-vektoriellen g-Kraftsensor über die Leitung 50 ein Ausgangssignal, das Informationen über die jeweilige g-Kraft in den drei Richtungen des Raumes umfasst. In Abhängigkeit von diesem Ausgangssignal erzeugt die Elektronikeinheit 40 Stellsignale zur Druckbeaufschlagung des Druckpolsters 6 über das Regelventil 36 bzw. zur Druckentlastung des Druckpolsters 6 über das Auslassventil 46. Die Drucksensoren 26 der Druckpolster 5, 6, 7, 8 liefern über die Leitung 52 ein den jeweiligen Druck im Druckpolster 6 anzeigendes Drucksignal zur elektronischen Regeleinheit 40. Ein Abstandssensor 56 kann vorgesehen sein, um über die Leitung 54 ein Abstandssignal an die Elektronikeinheit 40 zu liefern, das die tatsächlich erfolgte Ausdehnung des Druckpolsters 6 nach Anlegen eines Druckes über das Regelventil 36 anzeigt.

Es wird darauf hingewiesen, dass die vorderen Druckpolster 5, 6 innenseitig jeweils Einbuchtungen 28 in den Versteifungselementen 18, 20 aufweisen können, wie dies in der Figur 4 schematisch dargestellt ist., die von der Umhüllung bedeckt sein können und insbesondere ein schädliches Abdrücken der Arteria carotis communis sowie der Vena Ingularis verhindern. Solche Einbuchtungen vermindern bei der vorliegenden Einrichtung nicht die Abstützung der vorderen Druckpolster 5, 6 an den Unterkieferbereichen, weil sie oberseitig durch das Auflageteil 4 überdeckt werden.

Es wird darauf hingewiesen, dass die vorliegende Einrichtung auch zur Kompensation von Vibrationsbelastungen vorteilhaft anwendbar ist, wie sie beim Fliegen in Helikoptern auftreten und durch die Rotorblätter desselben verursacht werden. Derartige sinusförmige Vibrationen liegen im Frequenzbereich von 30 bis 100 Hz bzw. bei schallinduzierten oder durch GFK-Luftfahrzeugzellen übertragenen Frequenzen auch über 300 Hz mit einer ca. 0,15 oszillierenden g-Beschleunigung, bei einer länger andauernden Beschleunigung von etwa maximal 4g. Die Vibrationsbelastungen stammen entweder aus der Anregung über den Weg Sitz-Pilot auf den Kopf, aber auch bei höheren Frequenzen durch den Schalldruck des Rotors über Kabinendach-Luftstrecke direkt auf den Helm. Es können auch sogenannte PIOS-Schwingungen (Pilot Induced Oscillations) kompensiert werden.

## Patentansprüche

1. Einrichtung zum Entlasten der Halswirbelsäule, insbesondere beim Fliegen in einem Flugzeug, mit einer zwischen dem Kopf (1) eines Piloten und den Schultern desselben wirksamen Druckpolstereinrichtung, die wenigstens zwei vordere Druckpolster (5, 6) umfasst, deren vordere Endbereiche durch einen Spalt beabstandet sind, **dadurch gekennzeichnet, dass** ein am Helm (H) des Piloten angeordnetes Auflageteil (4) für das Kinn des Piloten vorgesehen ist und den Spalt zwischen den vorderen Endbereichen der vorderen Druckpolster (5, 6) überbrückend auf den Oberseiten der vorderen Druckpolster (5, 6) aufliegen und sich in Bezug auf diese verschieben kann, so dass der Kopf (1) des Piloten frei drehbar ist und sein Kinn nicht in den Spalt eintreten kann.

2. Einrichtung nach Anspruch 1, **dadurch gekennzeichnet, dass** die vorderen Druckpolster (5, 6) am Randbereich einer Öffnung (12) einer auf dem Nacken- und Schulterbereich des Piloten aufliegenden Auflageschale (10) befestigt sind, derart, dass ihre Oberseiten den seitlichen Unterkieferbereichen des Piloten zugewandt sind, und dass das Auflageteil (4) auf den Oberseiten gleitbar gelagert ist.

3. Einrichtung nach Anspruch 1 oder 2, **dadurch gekennzeichnet, dass** das Auflageteil (4) an Kinngurten (22, 24) des Helmes (H) befestigt ist.

4. Einrichtung nach einem der Ansprüche 1 bis 3, **dadurch gekennzeichnet, dass** die vorderen Druckpolster (5, 6) an ihren Oberseiten integrierte Versteifungselemente (18, 20) aufweisen, auf denen sich das Auflageteil (4) abstützen kann.

5. Einrichtung nach einem der Ansprüche 1 bis 4, **dadurch gekennzeichnet, dass** das Auflageteil (4) einen vorderen Bereich (4') aufweist, der den Spalt zwischen den vorderen Druckpolstern (5, 6) überbrückt, und dass an jedem Ende des vorderen Bereiches (4') ein Seitenbereich angeordnet ist, der sich ausgehend von dem vorderen Bereich (4') schräg nach außen erstreckt.

6. Einrichtung nach Anspruch 5, **dadurch gekennzeichnet, dass** sich die freien Endbereiche (4") der seitlichen Bereiche aus der Ebene Seitenbereiche schräg nach oben von den vorderen Druckpolster (5, 6) weg erstrecken.

7. Einrichtung nach einem der Ansprüche 1 bis 6, **dadurch gekennzeichnet, dass** die vorderen Druckpolster (5, 6) und hintere Druckpolster (7, 8) am Randbereich der Öffnung (12) der Auflageschale (10) angeordnet sind, wobei sich die hinteren Druckpolster (7, 8) oberseitig an der Hinterhauptschuppe des Kopfes (1) des Piloten abstützen.

8. Einrichtung nach Anspruch 7, **dadurch gekennzeichnet, dass** die Versteifungselemente (18, 20) der vorderen Druckpolster (5, 6) innenseitig jeweils wenigstens eine Einbuchtung (28) aufweisen, durch die ein Abdrücken wenigstens einer Arterie verhinderbar ist.

9. Einrichtung nach einem der Ansprüche 1 bis 8, **dadurch gekennzeichnet, dass** der Helm (H) über Kinngurtteile (22, 24) mit dem Auflageteil (4) verbunden ist.

10. Einrichtung nach Anspruch 9, **dadurch gekennzeichnet, dass** die Kinngurtteile (22, 24) an den Seitenbereichen des Auflageteiles (4) befestigt sind.

11. Einrichtung nach einem der Ansprüche 2 bis 10, **dadurch gekennzeichnet, dass** die Auflageschale (10) vorderseitig eine nach unten, von den vorderen Druckpolstern (5, 6) weg verlaufende schlitzförmige Öffnung (14) aufweist, die zum Anlegen der Auflageschale (10) öffenbar und durch eine Schließeinrichtung (16) verschließbar ist.

12. Einrichtung nach Anspruch 11, **dadurch gekennzeichnet, dass** sich die schlitzförmige Öffnung (14) ausgehend von dem Spalt erstreckt.

## Claims

1. A device for supporting the cervical vertebral column, especially during a flight in an aircraft, having a pressure pad device operative between the head (1) of a pilot and the shoulders of said pilot, which comprises at least two front pressure pads (5, 6), the front end areas of which are distanced from each other by a gap,
**characterised in that** a support component (4) disposed on the pilot's helmet (H) is provided for the pilot's chin and can lie on the upper surfaces of the pressure pads (5, 6) and can move in relation thereto, whilst bridging the gap between the front end areas of the front pressure pads (5, 6), so that the pilot's head (1) can turn freely and his chin cannot enter the gap.

2. A device according to Claim 1,
**characterised in that** the front pressure pads (5, 6) are fastened to the edge area of an opening (12) of a support shell (10) lying on the pilot's neck and shoulder area in such a manner that its upper surfaces face the lateral submaxillary areas of the pilot and the support component (4) is mounted to slide on the upper surfaces.

3. A device according to Claim 1 or 2,
**characterised in that** the support component (4) is fastened to chin straps (22, 24) of the helmet (H).

4. A device according to one of Claims 1 to 3,
**characterised in that** the upper surfaces of the front pressure pads (5, 6) comprise integrated strengthening components (18, 20) on which the support component (4) can be supported.

5. A device according to one of Claims 1 to 4,
**characterised in that** the support component (4) comprises a front area (4') bridging the gap between the front pressure pads (5, 6),
and **in that** at each end of the front area (4') there is disposed a side area, which extends obliquely outwards from the front area (4').

6. A device according to Claim 5,
**characterised in that** the free end areas (4") of the side areas extend out of the plane of the side areas obliquely upwards away from the front pressure pads (5, 6).

7. A device according to one of Claims 1 to 6,
**characterised in that** the front pressure pads (5, 6) and the rear pressure pads (7, 8) are disposed at the edge area of the opening (12) of the support shell (10), with the surface of the rear pressure pads (7, 8) being supported on the squamous part of the occipital bone of the pilot's head (1).

8. A device according to Claim 7,
**characterised in that** the inside surfaces of the strengthening components (18, 20) of the front pressure pads (5, 6) in each case comprise at least one indentation (28) by which pressure on at least one artery can be prevented.

9. A device according to one of Claims 1 to 8,
**characterised in that** the helmet (H) is connected to the support component (4) by chin strap components (22, 24).

10. A device according to Claim 9,
**characterised in that** the chin strap components (22, 24) are fastened to the side areas of the support component (4).

11. A device according to one of Claims 2 to 10,
**characterised in that** the front surface of the support shell (10) comprises a slit-shaped opening (14) that extends downwards away from the front pressure pads (5, 6) and that can be opened for putting on the support shell (10) and can be locked by a locking mechanism (16).

12. A device according to Claim 11,
**characterised in that** the slit-shaped opening (14) extends from the gap.

## Revendications

1. Dispositif pour soulager la colonne cervicale, plus particulièrement lors du pilotage d'un avion, comportant un système de coussinets d'appui (5, 6) qui agit entre la tête (1) d'un pilote et les épaules de celui-ci et comprend au moins deux coussinets d'appui antérieurs (5, 6), dont les parties d'extrémité sont séparées par un espace, **caractérisé en ce qu'**il est prévu, sur le casque (H) du pilote, un élément de maintien (4) pour le menton du pilote, qui ponte l'espace entre les parties d'extrémité des coussinets d'appui antérieurs (5, 6), prend appui sur les faces supérieures des coussinets antérieurs (5, 6) et peut se déplacer par rapport à ceux-ci d'une manière telle que la tête (1) du pilote puisse tourner librement et que son menton ne puisse pas s'engager dans l'espace.

2. Dispositif selon la revendication 1, **caractérisé en ce que** les coussinets d'appui antérieurs (5, 6) sont fixés dans la région du bord d'une ouverture (12) d'une coque de maintien (10) en appui sur la région de la nuque et des épaules du pilote de manière telle que leur face supérieure soit tournée vers le maxillaire inférieur du pilote et que l'élément de maintien (4) glisse sur les faces supérieures.

3. Dispositif selon la revendication 1 ou 2, **caractérisé en ce que** l'élément de maintien (4) est fixé à des sangles mentonnières (22, 24) du casque (H).

4. Dispositif selon une des revendications 1 à 3, **caractérisé en ce que** les coussinets d'appui antérieurs (5, 6) présentent sur leur face supérieure des éléments raidisseurs (18, 20) sur lesquels l'élément de maintien (4) peut prendre appui.

5. Dispositif selon une des revendications 1 à 4, **caractérisé en ce que** l'élément de maintien (4) présente une partie antérieure (4'), qui ponte l'espace entre les coussinets d'appui antérieurs (5, 6), et qu'à chaque extrémité de la partie antérieure (4') est disposée une partie latérale qui s'étend depuis la partie antérieure (4') en biais vers l'extérieur.

6. Dispositif selon la revendication 5, **caractérisé en ce que** les extrémités libres (4") des parties latérales s'étendent en biais vers le haut à partir du plan des parties latérales, dans la direction opposée au coussinets d'appui antérieurs (5, 6).

7. Dispositif selon une des revendications 1 à 6, **caractérisé en ce que** les coussinets d'appui antérieurs (5, 6) et les coussinets d'appui postérieurs (7, 8) sont disposés dans la région du bord de l'ouverture (12) de la coque de maintien (10), les coussinets d'appui postérieurs (7, 8) prenant appui côté supérieur sur l'os occipital de la tête (1) du pilote.

8. Dispositif selon la revendication 7, **caractérisé en ce que** les éléments raidisseurs (18, 20) des coussinets d'appui antérieurs (5, 6) côté intérieur présentent chacun au moins un évidement (28) qui permet d'éviter une compression d'au moins une artère.

9. Dispositif selon une des revendications 1 à 8, **caractérisé en ce que** le casque (H) est relié à l'élément d'appui (4) par des sangles de menton (22, 24).

10. Dispositif selon la revendication 9, **caractérisé en ce que** les sangles de menton (22, 24) sont fixées aux parties latérales l'élément de maintien (4).

11. Dispositif selon une des revendications 2 à 10, **caractérisé en ce que** la coque de maintien (10), sur le devant, présente une ouverture (14) en forme de fente, qui s'étend vers le bas à partir des coussinets d'appui antérieurs (5, 6) et peut être ouverte aux fins de mise en place de la coque de maintien (10) et fermée au moyen d'un dispositif de fermeture (16).

12. Dispositif selon la revendication 11, **caractérisé en ce que** l'ouverture (14) en forme de fente s'étend à partir de l'espace entre les coussinets.
